# EUROPEAN PATENT APPLICATION

(11) **EP 3 977 990 A1**
(43) Date of publication of application: **06.04.2022**
(21) Application number: 20815314.8
(22) Date of filing: 21.05.2020
(51) Int. Cl.: A61K 31/198, A61K 31/405, A61P 11/06, A61P 27/02, A23L 33/175

(54) **COMPOSITION FOR PREVENTING OR TREATING ASTHMA, RHINITIS OR CONJUNCTIVITIS, COMPRISING N-ACYL AMINO ACID AS ACTIVE INGREDIENT**

(30) Priority: 24.05.2019 KR 20190060932
(71) Applicant: Stemdr Inc., Jeonju-si, Jeollabuk-do 54858 (KR)
(72) Inventor: HAN, Myung-Kwan, Jeonju-si Jeollabuk-do 54955 (KR); LEE, Kwangho, Daejeon 34114 (KR)
(74) Representative: V.O.
(86) International application number: PCT/KR2020/006651
(87) International publication number: WO 2020/242133

(57) **Abstract**

The present disclosure relates to a composition for preventing, alleviating or treating asthma, rhinitis and/or conjunctivitis using an N-acylamino acid, which has almost no side effect on the human body. The composition of the present disclosure has an excellent effect of alleviating the symptoms of asthma by exhibiting alleviation effects on both inflammatory response in the airway and airway fibrosis, which are major clinical symptoms of asthma, and thus can be effectively used in drugs or foods for alleviating asthma. In addition, it can be usefully used in drugs or foods for alleviating rhinitis and conjunctivitis since it significantly alleviates the clinical symptoms of rhinitis and conjunctivitis.

## Description

### [Technical Field]

The present disclosure relates to a composition for preventing or treating asthma, rhinitis or conjunctivitis, which contains an N-acylamino acid as an active ingredient.

### [Background Art]

Asthma is an inflammatory disease of the respiratory organ caused by genetic and environmental factors, characterized by a condition where the bronchi in the lungs are hypersentitized, e.g., frequent shortness of breath, wheezing and severe coughing caused by narrowing of the bronchi. Recently, the incidence rate is increasing due to environmental pollution and increased exposure to chemicals. Although asthma occurs in all age groups, about 30% occurs in childhood. It is one of major diseases which develops into a chronic respiratory inflammatory disease and leads to frequent hospitalization and low quality of life (Eur Respir J 2001; 17(5): 881-6).

Rhinitis is a disease characterized by inflammatory responses of the nasal mucous membrane, and can be largely classified into allergic rhinitis and non-allergic rhinitis. Allergic rhinitis causes such symptoms as sneezing, runny nose, stuffy nose and swelling of mucous cells as a result of abnormal response of specific immunoglobulins (IgE, IgM, etc.). Non-allergic rhinitis includes infectious rhinitis caused by bacterial or fungal infection, in addition to coryza due to viral infection, vasomotor rhinitis that may occur due to the autonomous nervous system dysfunction of the nasal mucosa, rhinitis caused by improper medication, physical conditions such as cold temperature, etc., food-induced rhinitis, rhinitis caused by anatomical abnormality of the nasal structure, or the like.

The incidence of allergic rhinitis is increasing consistently around the world. In the US, allergic rhinitis has cost over 1.9 billion dollars in direct medical expenses (expenses for outpatients, prescriptions and emergency room visits) in the 20th century, and further expenses due to complications have reached 4 billion dollars. According to the 2011 National Health Insurance Statistical Yearbook, the number of Korean patients with allergic rhinitis has increased rapidly from 2 million in 2000 to 5.6 million in 2011 (from rank 14 to 5), and the expenditure by the National Health Insurance Service for allergic rhinitis ranked 4th among over 500 diseases. It is expected that the social cost spent on allergic rhinitis will increase further, and there is increasing need for the methods for treating or alleviating allergic rhinitis.

Conjunctivitis is a disease caused by the allergic response of the conjunctiva. It is often accompanied by allergic diseases such as hay fever or allergic rhinitis. Being a representative seasonal disease, the symptoms become severe in spring and are alleviated in fall or winter. It occurs frequently in individuals with allergic constitution. It usually begins before puberty and recurs for 5-10 years. Afterwards, the incidence decreases and the symptoms are alleviated. However, it is known to occur in people of all ages and both sexes regardless of season or constitution, due to the continued unusually hot weather. Its main causes are animal hair, dust, pollen, house dust mites, etc., and it has been recently known that pollutants such as exhaust gases from automobiles or chemical dusts also have great effects. General symptoms include severe eye irritation, severe redness, eyelid edema and increased production of tears, and the symptoms may be aggravated by rubbing. In addition, scratching or burning pain, foreign body sensation, sticky discharge or subconjunctival hemorrhage may occur.

The treatment of asthma depends mainly on medication. In general, anti-inflammatory agents (oral and inhaled steroids), bronchodilators (theophylline), antileukotriens, etc. are used (Clin Exp Allergy. 2012 42: 650-8).

For allergic rhinitis, medication of antihistamines is recommended to reduce symptoms. However, high doses of antihistamines may induce drowsiness or vertigo, and long-term medication may aggravate the symptoms on the contrary by inducing hypersecretion of histamine.

Glucocorticoid-based steroidal antiinflammatory drugs, which are representative therapeutic agents for conjunctivitis, are commonly used as antiinflammatory drugs. They exhibit superior efficacy for rheumatoid arthritis, etc. and inhibit or prevent various inflammatory responses caused by various stimuli such as radiation and mechanical, chemical, infectious or immunological factors.

Steroids are drugs that are the most widely used for allergic inflammatory diseases including asthma, rhinitis and conjunctivitis at present. For asthma, it is known that acute asthma is controlled relatively well with steroids. However, because chronic asthma and severe asthma with very severe symptoms do not respond to steroids, they cause long-lasting pains in many patients having such symptoms, significantly deteriorate the quality of life and even lead to death (Clin Exp Allergy. 2012 42: 650-8).

The problem of using of steroids is that they cause severe side effects. They cause fatal side effects such as increased risk of microbial infection or cancer by depressing immune response and induce dysfunction of skin, muscles, bone, eyes, nervous system, endocrine system, cardiovascular system, immune system, digestive organs, etc. and other diseases. Therefore, their use is very limited (N Engl J Med. 2005, 353: 1711-23).

Accordingly, the development of safe drugs for inhibiting allergic inflammation that can replace steroids is one of the most important and urgent issues that should be solved by modern medicine. In this regard, non-steroidal antiinflammatory drugs (NSAIDs) have been developed by researchers to overcome the side effects of steroids. A representative example is aspirin. Aspirin is an inhibitor of cyclooxygenase (COX), which is an enzyme involved in the synthesis of prostaglandin from arachidonic acid by the action of cytosolic phospholipase A₂ (cPLA₂). Aside from aspirin, many NSAIDs (indomethacin, ibuprofen, celecoxib, rofecoxib, etc.) are COX inhibitors. However, these NSAIDs are not used for treatment of allergic inflammatory diseases because their antiinflammatory action is weaker as compared to steroids. In addition, although scientists have developed the inhibitors of p38 mitogen-activated protein kinase and cPLA₂, which are inflammatory response-inducing enzymes, from long ago, none is used clinically due to side effects *(*Bioorg Med Chem 2008; 16: 1345-58).

Therefore, the development of drugs that can "supplement" or "replace" steroids will make an epoch-making contribution to the treatment of various allergic inflammatory diseases including asthma, and the development of such substances is keenly needed.

### [Disclosure]

### [Technical Problem]

The inventors of the present disclosure have made consistent efforts to develop a drug exhibiting superior effect of alleviating asthma, rhinitis and conjunctivitis without side effects. As a result, they have identified that the administration of an N-acylamino acid has an excellent effect of inhibiting asthma, rhinitis and conjunctivitis, and have completed the present disclosure.

The present disclosure is directed to providing a composition for preventing, alleviating or treating asthma, which contains an N-acylamino acid as an active ingredient.

The present disclosure is also directed to providing a composition for preventing, alleviating or treating rhinitis, which contains an N-acylamino acid as an active ingredient.

The present disclosure is also directed to providing a composition for preventing, alleviating or treating conjunctivitis, which contains an N-acylamino acid as an active ingredient.

Other purposes and advantages of the present disclosure will become more apparent by the following detailed description, claims and drawings.

### [Technical Solution]

In an aspect of the present disclosure, the present disclosure provides a composition for preventing, alleviating or treating asthma, rhinitis or conjunctivitis, which contains an N-acylamino acid as an active ingredient.

In another aspect of the present disclosure, the present disclosure provides a method for preventing, alleviating or treating asthma, rhinitis or conjunctivitis, which includes a step of administering an effective amount of an N-acylamino acid to a subject.

In another aspect of the present disclosure, the present disclosure provides a use of an N-acylamino acid for preventing, alleviating or treating asthma, rhinitis or conjunctivitis.

In the present disclosure, the asthma is a disease characterized by a condition where the bronchi in the lungs are hypersentitized, with such symptoms as shortness of breath, wheezing and severe coughing caused by narrowing of the bronchi. It is an allergic disease caused by the allergic inflammation of the bronchi.

In the present disclosure, the rhinitis is a disease characterized by inflammatory responses of the nasal mucous membrane, and can be largely classified into allergic rhinitis and non-allergic rhinitis. Allergic rhinitis is characterized by the hypersensitivity of the nasal mucosa to specific substances. It refers to a disease wherein, after exposure of the nasal mucosa to allergy-inducing substances (antigens), various inflammatory cells mediated by IgE antibody including mast cells and eosinophils are recruited to the stimulation site and inflammatory responses occur due to various mediators. Non-allergic rhinitis includes infectious rhinitis caused by bacterial or fungal infection, in addition to coryza due to viral infection, vasomotor rhinitis that may occur due to the autonomous nervous system dysfunction of the nasal mucosa, rhinitis caused by improper medication, physical conditions such as cold temperature, etc., food-induced rhinitis, rhinitis caused by anatomical abnormality of the nasal structure, or the like.

In the present disclosure, the conjunctivitis refers to conjunctivitis caused by topical stimulation. It varies from light congestion of the conjunctiva to eyelid swelling and conjunctival edema. In particular, allergic conjunctivitis is reacted with genetic factors and shows immediate allergy.

The N-acylamino acid of the present disclosure is specifically N-acylalanine or N-acyltryptophan, more specifically N-acyl-L-alanine or N-acyl-L-tryptophan.

The N-acyl-L-alanine or N-acyl-L-tryptophan is alanine or tryptophan in which an α-amino group is acylated. It may be synthesized by an organic synthesis method or may be purchased commercially.

In the present specification, the term "acyl" or "acyl group" is not specially limited and refers to a moiety obtained by removal of the OH of a carboxyl group of a carboxylic acid. It is generally represented by RCO. R is one or more substituent that may be bonded to CO without limitation. When the R is an aromatic moiety, it is particularly referred to "aroyl", but it is also an acyl group. Examples of the acyl include formyl (HCO-), acetyl (CH₃CO-), propionyl (C₂H₅CO-), butyryl (C₃H₇CO-), valeryl (C₄H₉CO-), pentanoyl (CH₃(CH₂)₃CO-), palmitoyl (C₁₅H₃₁CO-), stearoyl (C₁₇H₃₃CO-), oleoyl (C₁₇H₃₁CO-), oxalyl (-CO-CO-), malonyl (-COCH₂CO-), succinyl (-CO(CH₂)₂CO-), benzoyl (C₆H₅CO-), toluoyl (CH₃-C₆H₄-CO-), salicyloyl (HO-C₆H₄-CO-), cinnamoyl (C₆H₅CH=CHCO-), naphthoyl (C₁₀H₇CO-), phthaloyl (CO-C₆H₄-CO-), furoyl (C₅H₃O₂-), undecanoyl (CH₃(CH₂)₉CO-) and docosenoyl obtained by removing an OH group from docosenoic acid, although not being limited thereto.

Specifically, the N-acylalanine may be N-acetylalanine or N-oleoylalanine, although not being limited thereto.

Specifically, the N-acyltryptophan may be N-acetyltryptophan or N-oleoyltryptophan, although not being limited thereto.

Specifically, the N-acyl-L-alanine may be N-acetyl-L-alanine or N-oleoyl-L-alanine, although not being limited thereto.

Specifically, the N-acyl-L-tryptophan may be N-acetyl-L-tryptophan or N-oleoyl-L-tryptophan, although not being limited thereto.

In the present specification, the term "containing as an active ingredient" or "effective amount" means that the N-acylamino acid is contained in an amount sufficient to achieve its effect or activity. In a specific exemplary embodiment of the present disclosure, the composition of the present disclosure contains the N-acylamino acid in an amount of, for example, 10 µg/kg or more, specifically 0.1 mg/kg or more, more specifically 1 mg/kg or more, further more specifically 10 mg/kg or more. Since the N-acylamino acid has almost no side effect on the human body even when it is administered in excess amount, the upper limit of the amount of the N-acylamino acid in the composition of the present disclosure may be determined adequately by those skilled in the art.

The N-acylamino acid used in the composition of the present disclosure as an active ingredient may include not only the compound on its own but also a pharmaceutically, sitologically or cosmetically acceptable salt, hydrate, solvate or prodrug thereof.

In the present specification, the term "pharmaceutically acceptable salt", "sitologically acceptable salt" or "cosmetically acceptable salt" refers to a form of a compound which does not induce severe irritation in an organism to which the compound is administered and does not negatively affect the biological activity and physiological properties of the compound. The pharmaceutically, sitologically or cosmetically acceptable salt may be obtained by reacting the compound of the present disclosure with an inorganic acid such as hydrochloric acid, bromic acid, sulfuric acid, nitric acid, phosphoric acid, etc., or an organic acid such as sulfonic acid, e.g., methanesulfonic acid, ethanesulfonic acid, p-toluenesulfonic acid, etc., tartaric acid, formic acid, citric acid, acetic acid, trichloroacetic acid, trifluoroacetic acid, capric acid, isobutyric acid, malonic acid, succinic acid, phthalic acid, gluconic acid, benzoic acid, lactic acid, fumaric acid, maleic acid, salicylic acid, etc. In addition, an ammonium salt, an alkali metal salt such as a sodium or potassium salt, an alkaline earth metal salt such as a calcium or magnesium salt, a salt of an organic base such as dicyclohexylamine, N-methyl-D-glucamine, tris(hydroxymethyl)methylamine, etc., or an amino acid of arginine, lysine, etc. may be obtained by reacting the compound of the present disclosure with a base, although not being limited thereto.

In the present specification, the term "pharmaceutically acceptable hydrate", "sitologically acceptable hydrate" or "cosmetically acceptable hydrate" refers to a hydrate of the N-acylamino acid having a desired pharmacological effect. The term "pharmaceutically acceptable solvate", "sitologically acceptable solvate" or "cosmetically acceptable solvate" refers to a solvate of the N-acylamino acid having a desired pharmacological effect. The hydrate and the solvate may also be prepared using the acids described above and are included in the pharmaceutically, sitologically or cosmetically acceptable salt in a broad sense.

In the present specification, the term "pharmaceutically acceptable prodrug", "sitologically acceptable prodrug" or "cosmetically acceptable prodrug" refers to a derivative of the N-acylamino acid which requires biological conversion to exhibit the pharmacological effect of the N-acylamino acid. The prodrug is prepared to improve chemical stability, patient compliance, biological availability, organ selectivity or convenience of preparation, prolong the duration of action, and reduce side effects. The prodrug of the present disclosure may be prepared easily using the N-acylamino acid according to a method commonly used in the art (e.g., Burger's Medicinal Chemistry and Drug Chemistry, 5th ed., 1: 172-178 and 949-982 (1995)).

In a specific exemplary embodiment of the present disclosure, the composition of the present disclosure is a pharmaceutical composition.

In a specific exemplary embodiment of the present disclosure, the pharmaceutical composition of the present disclosure contains a pharmaceutically acceptable carrier.

The pharmaceutically acceptable carrier contained in the pharmaceutical composition of the present disclosure may include, as those commonly used for preparation, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, etc., although not being limited thereto. The pharmaceutical composition of the present disclosure may further contain, in addition to the above-described ingredients, a lubricant, a wetting agent, a sweetener, a flavorant, an emulsifier, a suspending agent, a preservative, etc. Suitable pharmaceutically acceptable carriers and preparations are described in detail in Remington's Pharmaceutical Sciences (19th ed., 1995).

The pharmaceutical composition of the present disclosure may be administered orally or parenterally. For parenteral administration, it may be administered nasally, periocularly, intravenously, subcutaneously, intramuscularly, intraperitoneally, transdermally, rectally, intrauterinely, intracranially, etc.

An adequate administration dosage of the pharmaceutical composition of the present disclosure varies depending on such factors as preparation method, mode of administration, the age, body weight, sex, pathological condition and diet of a patient, administration time, administration route, excretion rate and response sensitivity, and an ordinarily skilled physician can easily determine and prescribe an administration dosage effective for the desired treatment or prevention. In a specific exemplary embodiment of the present disclosure, a daily administration dosage of the pharmaceutical composition of the present disclosure is 10 µg/kg to 1,000 mg/kg.

The pharmaceutical composition of the present disclosure may be prepared into a single-dosage form or a multiple-dosage form using a pharmaceutically acceptable carrier and/or excipient according to a method that can be easily carried out by those having ordinary knowledge in the art to which the present disclosure belongs. The formulation may be in the form of a solution in an oily or aqueous medium, a suspension, an emulsion, an extract, a powder, a granule, a tablet or a capsule, and may further contain a dispersant or a stabilizer.

The pharmaceutical composition of the present disclosure may be prepared into a formulation for external application to skin, aerosol, a spray, an eye drop, an oral formulation or an injection.

The pharmaceutical composition of the present disclosure may be used for prevention or treatment of asthma, rhinitis or conjunctivitis either alone or in combination with surgery, radiation therapy, hormone therapy, chemotherapy or a method using a biological response modifier.

The pharmaceutical composition of the present disclosure may be used for human or animals.

In the present specification, the term "subject" refers to a human or an animal requiring administration of the N-acylamino acid.

In the present specification, the term "prevention" refers to any action of inhibiting asthma, rhinitis or conjunctivitis or delaying the progress thereof by administering the composition of the present disclosure.

In the present specification, the term "treatment" refers to any action of improving or favorably changing asthma, rhinitis or conjunctivitis by administering the composition of the present disclosure.

In a specific exemplary embodiment of the present disclosure, the composition of the present disclosure is a food composition.

The food composition according to the present disclosure may be used as a functional food or may be added to various foods. The foods to which the composition of the present disclosure may be added include, for example, beverages, alcohol beverages, confectionery, diet bars, dairy products, meat, chocolate, pizza, bread, ramen, other noodles, gums, ice creams, vitamin complexes, health supplements, etc.

The food composition of the present disclosure may further contain, in addition to the N-acylamino acid as an active ingredient, ingredients commonly added when preparing foods. For example, it contains a protein, a carbohydrate, a fat, a nutrient, a condiment and a flavorant. Examples of the carbohydrate include common sugars such as a monosaccharide, e.g., glucose, fructose, etc., a disaccharide, e.g., maltose, sucrose, oligosaccharide, etc., a polysaccharide, e.g., dextrin, cyclodextrin, etc., and sugar alcohols such as xylitol, sorbitol, erythritol, etc. As the flavorant, a natural flavorant (thaumatin or stevia extract (e.g., rebaudioside A, glycyrrhizin, etc.)) or a synthetic flavorant (saccharin, aspartame, etc.) may be used. For example, when the food composition of the present disclosure is prepared into a drink or a beverage, it may further contain, in addition to the N-acylamino acid, citric acid, fructose syrup, sucrose, glucose, acetic acid, malic acid, fruit juice, plant extract, etc.

In addition, the food composition of the present disclosure may further contain various nutrients, vitamins, electrolytes, flavorants, colorants, pectic acid and its salts, alginic acid and its salts, organic acids, protective colloidal thickeners, pH control agents, stabilizers, antiseptics, glycerin, alcohols, carbonating agents used in carbonated beverages, etc. Furthermore, the composition of the present disclosure may contain a pulp used for preparing natural fruit juice, fruit juice beverages and vegetable beverages. These ingredients may be used either alone or in combination. The content of these additives is selected generally from a range of 0.01-10 parts by weight based on 100 parts by weight of the composition of the present disclosure, although it is not of great importance.

The present disclosure provides a functional health food as a food composition containing the N-acylamino acid or a sitologically acceptable salt thereof as an active ingredient. The functional health food refers to a food that provides the function of preventing and alleviating a disease, protecting the body, providing immunity, recovering after illness, preventing aging, etc., and should be unharmful to the human body after long-term intake. The functional health food is prepared into a capsule, a powder, a suspension, etc. by adding the N-acylamino acid to food materials such as beverages, teas, spices, gums, confectionery, etc. Although it provides particular advantages in health, it does not have side effects unlike pharmaceuticals even after long-term intake. The functional health food of the present disclosure is very useful because it can be taken routinely. The N-acylamino acid may be added to the functional health food in an amount not negatively affecting the intrinsic taste of the food without limitation. It may be added to the food in an amount of generally 0.01-50 wt%, specifically 0.1-20 wt%. For a functional health food in the form of a pill, a granule, a tablet or a capsule, it may be added in an amount of generally 0.1-100 wt%, specifically 0.5-80 wt%. In a specific exemplary embodiment, the functional health food of the present disclosure may be in the form of a pill, a tablet, a capsule or a beverage.

As used in the present specification, the term "alleviation" refers to any action of stopping the progression of or alleviating symptoms related with asthma, rhinitis or conjunctivitis by ingesting or administering the composition of the present disclosure.

The food composition of the present disclosure may be used as a food for human, a feed for animals, a feed additive, etc.

In a specific exemplary embodiment of the present disclosure, the composition of the present disclosure is a cosmetic composition.

When the composition of the present disclosure is prepared as a cosmetic composition, the composition of the present disclosure may further contain, in addition to the N-acyl amino acid, ingredients commonly used in cosmetic compositions, e.g., a common adjuvant such as an antioxidant, a stabilizer, a solubilizer, a vitamin, a pigment and a flavor, and a carrier. In addition, the composition of the present disclosure may be prepared by mixing the N-acylamino acid with a conventionally used agent for alleviating or pacifying asthma, rhinitis or conjunctivitis within a range not negatively affecting its action.

As the carrier, purified water, monohydric alcohols (ethanol or propyl alcohol), polyhydric alcohols (glycerol, 1,3-butylene glycol or propylene glycol), higher fatty acids (palmitic acid or linoleic acid), oils or fats (wheat germ oil, camellia oil, jojoba oil, olive oil, squalene, sunflower oil, macadamia nut oil, avocado oil, hydrogenated soybean lecithin or fatty acid glyceride), etc. may be used, although not being limited thereto. In addition, a surfactant, a sterilizer, an antioxidant, a UV absorbent, an antiphlogistic or a cooling agent may be added if necessary.

The surfactant may be selected from a group consisting of polyoxyethylene, hydrogenated castor oil, oleyl ether, polyoxyethylene monooleate, glyceryl monostearate, sorbitan monostearate, sorbitan, sucrose fatty acid ester, hexaglyceryl monolaurate, polyoxyethylene reduced lanolin, POE, glyceryl pyroglutamate, isostearate, diester, N-acetylglutamine and isostearyl ester.

The sterilizer may be selected from a group consisting of hinokitiol, triclosan, chlorhexidine gluconate, phenoxyethanol, resorcin, isopropylmethylphenol, azulene, salicylic acid and zinc pyrithione.

As the antioxidant, any of butylhydroxyanisole, gallic acid, propyl gallate and erythorbic acid may be used.

As the UV absorbent, any of benzophenones such as dihydroxybenzophenone, melanin, ethyl p-aminobenzoate, p-dimethylaminobenzoic acid 2-ethyl hexyl ester, cinoxate, p-methoxycinnamonic acid 2-ethyl hexyl ester, 2-(2-hydroxy-5-methylphenyl)benzotriazole, urocanic acid and metal oxide powder may be used.

Dipotassium glycyrrhizinate or allantoin may be used as the antiphlogistic, and capsicum tincture or 1-menthol may be used as the cooling agent.

The composition may be prepared into any formulation in which the N-acylamino acid may be mixed as an active ingredient. For example, it may be prepared into a tonic, a shampoo, a rinse, a hair conditioner, a hair spray, a powder, a gel, a cream, an essence, a lotion, a solgel, an emulsion, an oil, a wax, a spray, a mist, etc., although not being limited thereto.

### [Advantageous Effects]

The features and advantages of the present disclosure may be summarized as follows.
(i) The present disclosure provides a composition for preventing, alleviating or treating asthma, rhinitis or conjunctivitis using an N-acylamino acid.
(ii) The composition of the present disclosure may be safely and usefully used to alleviate or treat allergic diseases and asthma resulting from various causes without the concern of side effects.

### [Brief Description of Drawings]

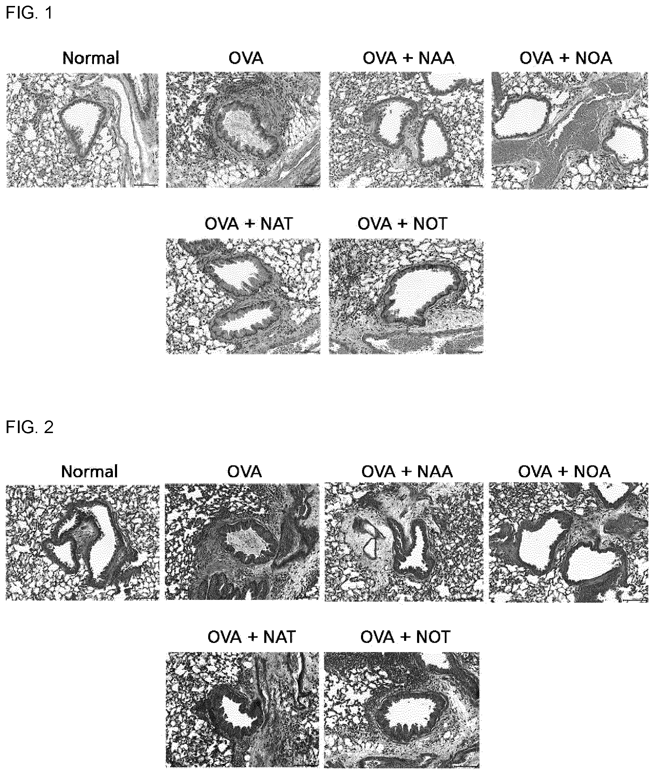

FIG. 1 shows H&E staining images of lung tissue showing the inhibition of inflammation by oral administration of N-acyl-L-alanine, N-oleoyl-L-alanine, N-acyl-L-tryptophan and N-oleoyl-L-tryptophan (OVA = ovalbumin, NAA = N-acetyl-L-alanine, NOA = N-oleoyl-L-alanine, NAT = N-acetyl-L-tryptophan, NOT = N-oleoyl-L -tryptophan).
FIG. 2 shows Masson's trichrome staining images of lung tissue showing the inhibition of bronchial fibrosis by oral administration of N-acyl-L-alanine, N-oleoyl-L-alanine, N-acyl-L-tryptophan and N-oleoyl-L-tryptophan (OVA = ovalbumin, NAA = N-acetyl-L-alanine, NOA = N-oleoyl-L-alanine, NAT = N-acetyl-L-tryptophan, NOT = N-oleoyl-L-tryptophan).

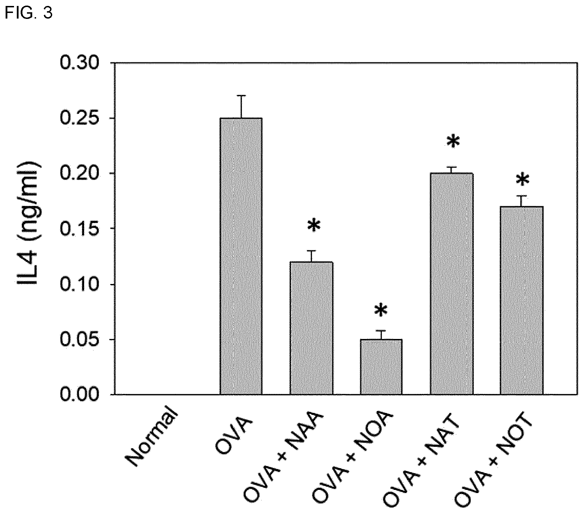

FIG. 3 shows the inhibition of Th2 cytokine (IL4) secretion by cells in the airway by N-acetyl-L-alanine, N-oleoyl-L-alanine, N-acetyl-L-tryptophan and N-oleoyl-L-tryptophan (*p < 0.05 vs ovalbumin group; OVA = ovalbumin, NAA = N-acetyl-L-alanine, NOA = N-oleoyl-L-alanine, NAT = N-acetyl-L-tryptophan, NOT = N-oleoyl-L -tryptophan).

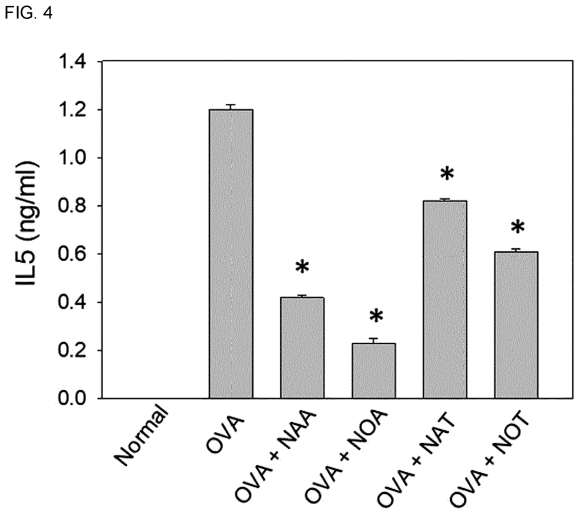

FIG. 4 shows the inhibition of Th2 cytokine (IL5) secretion by cells in the airway by N-acetyl-L-alanine, N-oleoyl-L-alanine, N-acetyl-L-tryptophan and N-oleoyl-L-tryptophan (*p < 0.05 vs ovalbumin group; OVA = ovalbumin, NAA = N-acetyl-L-alanine, NOA = N-oleoyl-L-alanine, NAT = N-acetyl-L-tryptophan, NOT = N-oleoyl-L -tryptophan).

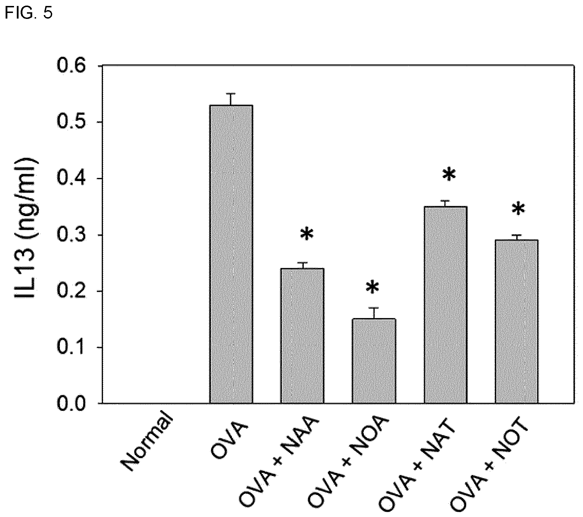

FIG. 5 shows the inhibition of Th2 cytokine (IL13) secretion by cells in the airway by N-acetyl-L-alanine, N-oleoyl-L-alanine, N-acetyl-L-tryptophan and N-oleoyl-L-tryptophan (*p < 0.05 vs ovalbumin group; OVA = ovalbumin, NAA = N-acetyl-L-alanine, NOA = N-oleoyl-L-alanine, NAT = N-acetyl-L-tryptophan, NOT = N-oleoyl-L -tryptophan).

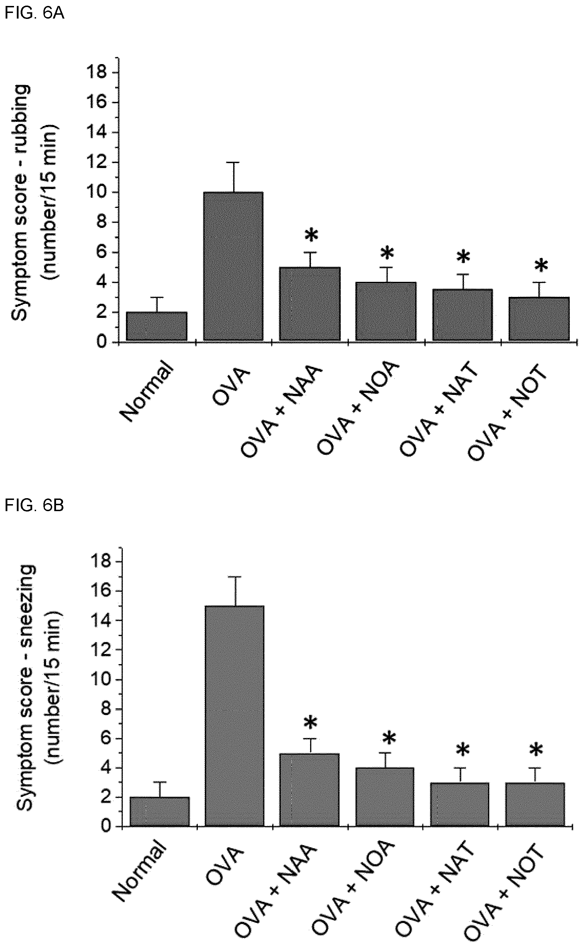

FIG. 6 shows the inhibition of rhinitic response by N-acetyl-L-alanine, N-oleoyl-L-alanine, N-acetyl-L-tryptophan and N-oleoyl-L-tryptophan (FIG. 6A: nasal rubbing, FIG. 6B: sneezing; *p < 0.05 vs ovalbumin group; OVA = ovalbumin, NAA = N-acetyl-L-alanine, NOA = N-oleoyl-L-alanine, NAT = N-acetyl-L-tryptophan, NOT = N-oleoyl-L -tryptophan).

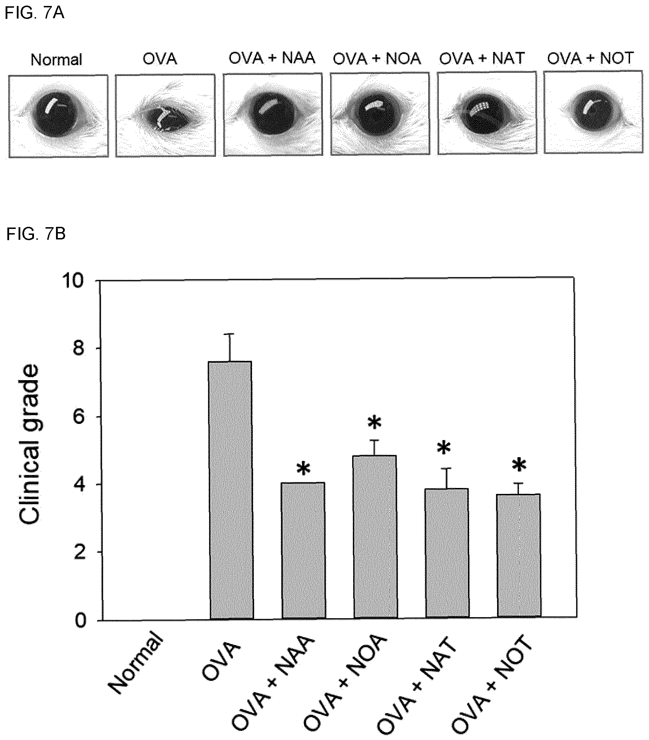

FIG. 7 shows the inhibition of allergic conjunctivitis by N-acetyl-L-alanine, N-oleoyl-L-alanine, N-acetyl-L-tryptophan and N-oleoyl-L-tryptophan (FIG. 7A: images of eye, FIG. 7B: clinical grade; *p < 0.05 vs ovalbumin group; OVA = ovalbumin, NAA = N-acetyl-L-alanine, NOA = N-oleoyl-L-alanine, NAT = N-acetyl-L-tryptophan, NOT = N-oleoyl-L-tryptophan).

### [Best Mode]

Hereinafter, the present disclosure is described in further detail through examples. These examples are provided only to illustrate the present disclosure more specifically, and it will be obvious to those having ordinary knowledge in the art that the scope of the present disclosure is not limited by the examples.

### Examples

### Preparation Example 1. Preparation of experimental animals

Specific pathogen-free BALB/c mice (6-week-old, male) were purchased from Doul Biotech (Osan, Korea). Experiment was started when the mice were 7- to 10-week old. The mice were housed in a laminar flow cabinet and were given water and feed ad libitum.

### Preparation Example 2. Preparation of drugs

As the drugs used in the present disclosure, N-acetyl-L-alanine and N-acetyl-L-tryptophan were purchased from Sigma (MO, USA), and N-oleoyl-L-alanine was purchased from Cayman (MI, USA). N-Oleoyltryptophan was synthesized as follows. First, for preparation of methyl oleoyl L-tryptophanate, a mixture of oleoic acid (2.36 mmol), L-tryptophan methyl ester hydrochloride (2.60 mmol), 1-ethyl-3-(3-dimethylaminopropyl)carbodiimide (EDCI) (2.60 mmol), hydroxybenzotriazole (HOBt) (2.60 mmol) and triethylamine (11.8 mmol) dissolved in dichloromethane was stirred at room temperature for 12 hours. The reaction mixture was concentrated, diluted with saturated NaHCO₃ solution, and then extracted 3 times with ethyl acetate. The extracted organic solvent layer was combined and washed with brine. After washing with 1 N HCI and then with brine, the organic solvent layer was dried on anhydrous MgSO₄, concentrated, and then purified by medium-pressure liquid chromatography (MPLC) using n-hexane and ethyl acetate. Yield was 73% and the data for the prepared methyl oleoyl L-tryptophanate are as follows. ¹H NMR (500 MHz, chloroform-d) δ 8.18 (s, 1H), 7.56 (dd, J = 7.9, 1.0 Hz, 1H), 7.39 (dt, J = 8.1, 0.9 Hz, 1H), 7.29 (s, 1H), 7.22 (ddd, *J* = 8.2, 7.1, 1.2 Hz, 1H), 7.14 (ddd, J = 8.0, 7.0, 1.0 Hz, 1H), 7.00 (d, *J* = 2.4 Hz, 1H), 5.99 (d, *J* = 7.9 Hz, 1H), 5.37 (qd, J = 4.1, 2.1 Hz, 2H), 5.00 (dt, J = 8.0, 5.3 Hz, 1H), 3.72 (s, 3H), 3.39-3.28 (m, 2H), 2.20-2.13 (m, 2H), 2.08-1.99 (m, 5H), 1.60 (t, *J = 7.4* Hz, 2H), 1.34-1.28 (m, 24H), 0.92-0.89 (m, 3H). LC-MS (ESI), calcd for C₃₀H₄₆N₂O₃ 482.4, found *m*/*z* 483.4 (M + H⁺). For preparation of N-oleoyltryptophan from the methyl oleoyl L-tryptophanate, a solution of the methyl oleoyl L-tryptophanate (0.37 mmol) dissolved in tetrahydrofuran was stirred at room temperature for 12 hours after adding NaOH (1.48 mmol) solution. The reaction mixture was extracted with dichloromethane after adding water. The aqueous layer was adjusted to pH 1 by adding 1 N HCI, and then extracted 3 times with ethyl acetate. The extracted organic solvent layer was dried on anhydrous MgSO₄, and then concentrated. Yield was 86%, and the data for the prepared N-oleoyltryptophan are as follows. ¹H NMR (500 MHz, chloroform-d) δ 8.22 (s, 1H), 7.61 (d, *J* = 7.9 Hz, 1H), 7.40 (dt, J = 8.1, 0.9 Hz, 1H), 7.23 (ddd, J = 8.1, 6.9, 1.2 Hz, 1H), 7.15 (ddd, J = 8.0, 7.1, 1.0 Hz, 1H), 7.08 (d, *J* = 2.4 Hz, 1H), 5.37 (qd, J = 5.3, 4.6, 2.3 Hz, 2H), 4.95 (dt, J = 7.3, 5.6 Hz, 1H), 3.45-3.33 (m, 2H), 2.17-2.10 (m, 2H), 1.59-1.51 (m, 2H), 1.38-1.26 (m, 26H), 0.91 (d, *J* = 6.8 Hz, 3H). C₂₉H₄₄N₂O₂ 468.3, found *m*/*z* 469.3 (M + H⁺).

### Preparation Example 3. Induction of asthma in mice

The mice were sensitized by intraperitoneally injecting 0.02 mg of ovalbumin (OVA) and alum (1 mg) (Sigma-Aldrich) as an immunologic adjuvant twice on days 0 and 14. For 4 days from day 28, the mice were placed in a plastic nebulizer container of a regular size and were made to inhale OVA in the form of an aerosol by spraying 5% OVA for 30 minutes (airway challenge).

### Preparation Example 4. Induction of rhinitis in mice

The mice were sensitized by intraperitoneally injecting 0.02 mg of ovalbumin (OVA) and alum (1 mg) (Sigma-Aldrich) as an immunologic adjuvant twice on days 0 and 14. For 7 days from day 21, the mice were topically stimulated by injecting 10 µL of a solution of 100 µg of OVA dissolved in 20 µL of phosphate-buffered saline into both nasal cavities, once a day. After the final injection of OVA (day 27), symptom score was recorded by summing the numbers of nasal rubbing and sneezing. The symptom score was recorded for 15 minutes, and the result was compared between the groups. The result is shown in FIG. 6.

### Preparation Example 5. Induction of conjunctivitis in mice

The mice were sensitized by intraperitoneally injecting 0.02 mg of ovalbumin (OVA) and alum (1.5 mg) (Sigma-Aldrich) as an immunologic adjuvant twice on days 0 and 7. For 12 days from day 14, conjunctivitis was induced by injecting 10 µL of a solution of 100 µg of OVA dissolved in 20 µL of phosphate-buffered saline into each eye, once a day.

### Preparation Example 6. Measurement of in inflammatory cells in bronchus

After anesthetizing the mouse and inserting a tube into the bronchus, the bronchus was washed with a buffer and cells were obtained therefrom. The cells were centrifuged, suspended at 1×10⁴ cells/100 µL, and fixed by centrifuging with a cytospin. Then, the number of eosinophils, monocytes, neutrophils and macrophages were measured by staining with Diff-Quik.

### Preparation Example 7. Staining of lung tissue

For histological assay, tissues taken out from the lung were fixed with formalin and embedded in paraffin. The embedded tissue was sliced to 4-µm thickness and subjected to 1) hematoxylin and eosin (H&E) staining for investigation of the degree of inflammation and 2) Masson's trichrome staining for investigation of the degree of pulmonary fibrosis.

### Preparation Example 8. Measurement of clinical grading of conjunctival edema and flare

24 hours after the final injection of OVA (day 25), the clinical grading of conjunctival edema and flare (redness) was assessed using a portable slit lamp (Carl Zeiss, Oberkochen, Germany). The assessment was performed in a blinded fashion 2 hours after OVA injection. Each clinical parameter was scored on a scale of 0 to 4+ (0 absent, 1+ minimal, 2+ mild, 3+ moderate, 4+ severe) as described in Merayo-Lloves, J., Calonge, M, and Foster, C.S. 1995. Experimental model of allergic conjunctivitis to ragweed in guinea pig. Curr. Eye Res. 14: 487-494. A negative control group was given no treatment.

### Example 1. Asthma-inhibiting effect of N-acetyl-L-alanine. N-oleoyl-L-alanine. N-acetyl-L-tryptophan and N-oleoyl-L-tryptophan

### Example 1-1. Inhibition of inflow of inflammatory cell into airway of asthma-induced mice by administration of N-acetyl-L-alanine. N-oleovl-L-alanine. N-acetyl-L-tryptophan and N-oleoyl-L-tryptophan

Neutrophils, macrophages, monocytes and eosinophils were observed as inflammatory cells in the airway. Each of N-acetyl-L-alanine, N-oleoyl-L-alanine, N-acetyl-L-tryptophan and N-oleoyl-L-tryptophan was dissolved in 10 mL of phosphate-buffered saline to 3.5 µmol/kg and was orally administered for 7 days from day 25, once a day. As seen from Table 1, the orally administration of N-acetyl-L-alanine, N-oleoyl-L-alanine, N-acetyl-L-tryptophan and N-oleoyl-L-tryptophan significantly inhibited the inflow of eosinophils into the airway.

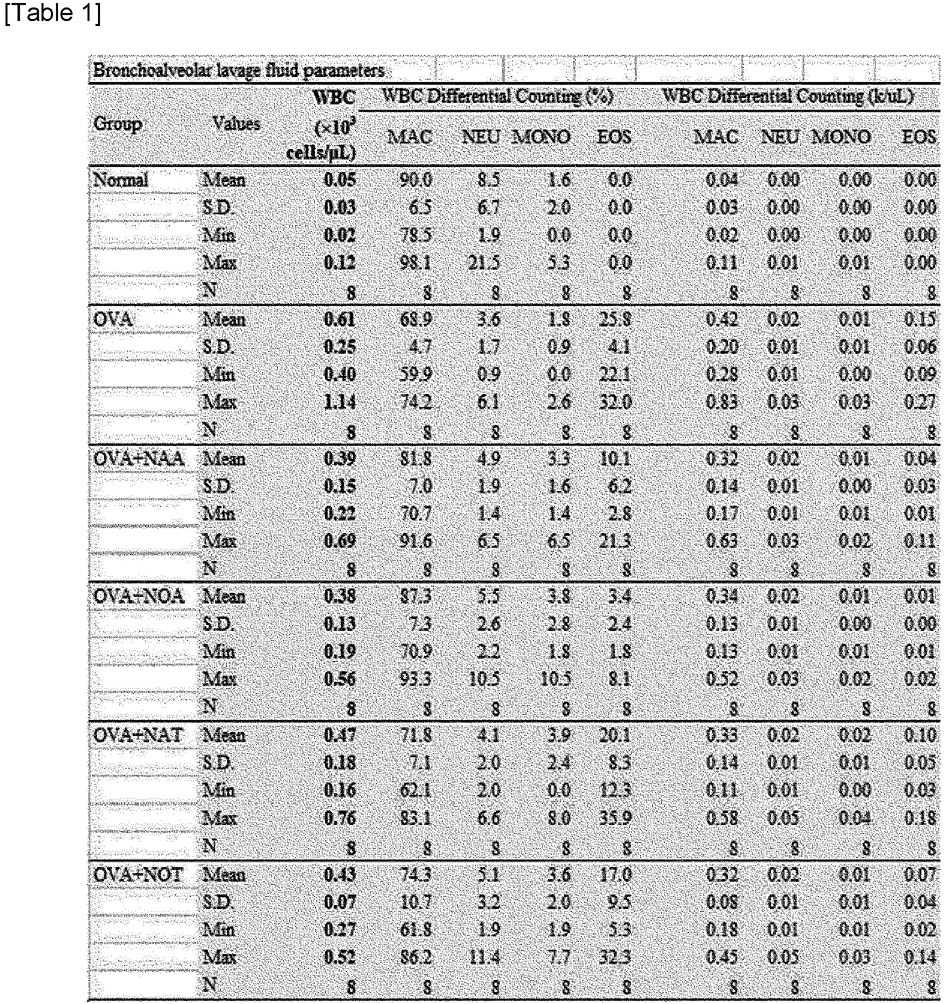

OVA = ovalbumin, NAA = N-acetyl-L-alanine, NOA = N-oleoyl-L-alanine, NAT = N-acetyl-L-tryptophan, NOT = N-oleoyl-L-tryptophan, MAC = macrophages, NEU = neutrophils, MONO = monocytes, EOS = eosinophils, N = number of mice, S.D. = standard deviation, Min = minimum value, Max = maximum value.

### Example 1-2. Investigation of degree of inflammation by H&E staining of lung tissue

Lung tissue was stained with H&E after oral administration of the drug for a week. While a normal group (normal) showed almost no inflammatory cells around the airway and an OVA-challenged group (OVA) showed many inflammatory cells gathered around the airway as well as narrowing of the bronchus and blood vessel formation around the bronchus (FIG. 1), the groups to which N-acetyl-L-alanine, N-oleoyl-L-alanine, N-acetyl-L-tryptophan or N-oleoyl-L-tryptophan was orally administered (OVA + NAA, OVA + NOA, OVA + NAT, OVA + NOT) showed remarkably inhibited inflammation. In particular, N-oleoyl-L-alanine showed the highest inhibition of inflammation (FIG. 1).

### Example 1-3. Measurement of cytokines in bronchial wash

The concentration of several cytokines (TNF-α, IL-4, IL-5, IL-13) in bronchial wash was measured using an ELISA kit (R&D Systems, Minneapolis, MN, USA).

### Example 2. Bronchial obstruction-inhibiting effect of N-acetyl-L-alanine. N-oleoyl-L-alanine. N-acetyl-L-tryptophan and N-oleoyl-L-tryptophan

Lung tissue was stained with Masson's trichrome after oral administration of the drug for a week. Whereas a normal group (normal) showed almost no fibrosis around the airway and an OVA-challenged group (OVA) showed a high degree of fibrosis around the airway (FIG. 2), the groups to which N-acetyl-L-alanine, N-oleoyl-L-alanine, N-acetyl-L-tryptophan or N-oleoyl-L-tryptophan was orally administered (OVA + NAA, OVA + NOA, OVA + NAT, OVA + NOT) showed inhibited fibrosis. In particular, N-oleoyl-L-alanine showed the highest inhibition (FIG. 2).

### Example 3. Inhibition of Th2 cytokine (IL4. IL5 and IL13) secretion in airway by N-acetyl-L-alanine, N-oleoyl-L-alanine. N-acetyl-L-tryptophan and N-oleoyl-L-tryptophan

Th2 cytokines, IL-4, IL-5 and IL-13, are known to be involved in airway inflammation, bronchial hypersensitivity and IgE antibody production mediated by eosinophils. 3.5 µmol/kg N-acetyl-L-alanine, N-oleoyl-L-alanine, N-acetyl-L-tryptophan or N-oleoyl-L-tryptophan dissolved in 10 mL of phosphate-buffered saline was orally administered once a day, for 7 days from day 25 after the induction of asthma, and the cytokines were measured. The oral administration of N-acetyl-L-alanine, N-oleoyl-L-alanine, N-acetyl-L-tryptophan and N-oleoyl-L-tryptophan significantly inhibited IL-4, IL-5 and IL-13 (FIG. 3, FIG. 4 and FIG. 5).

### Example 4. Rhinitic response-inhibiting effect of N-acetyl-L-alanine, N-oleoyl-L-alanine, N-acetyl-L-tryptophan and N-oleoyl-L-tryptophan

Ovalbumin (OVA) of Preparation Example 3 was used as an antigen to induce allergic rhinitis. After OVA challenging from day 21 to day 26, 10 µL of each of N-acetyl-L-alanine, N-oleoyl-L-alanine, N-acetyl-L-tryptophan and N-oleoyl-L-tryptophan dissolved in phosphate-buffered saline to 10 µM was intranasally administered 12 hours later. After the final injection of OVA (day 27), symptom score was recorded by summing the numbers of nasal rubbing and sneezing. The symptom score was recorded for 15 minutes, and the result was compared between the groups. The result is shown in FIG. 6. Whereas a normal group showed almost no nasal rubbing or sneezing and an OVA-challenged group showed remarkably increased nasal rubbing or sneezing (FIG. 6A and FIG. 6B), the groups to which N-acetyl-L-alanine, N-oleoyl-L-alanine, N-acetyl-L-tryptophan or N-oleoyl-L-tryptophan was intranasally administered showed remarkably decreased numbers of nasal rubbing and sneezing (FIG. 6A and FIG. 6B).

### Example 5. Conjunctivitis-inhibiting effect of N-acetyl-L-alanine. N-oleoyl-L-alanine, N-acetyl-L-tryptophan and N-oleoyl-L-tryptophan

Ovalbumin of Preparation Example 4 was used as an antigen to induce conjunctivitis. After challenging 100 µg of ovalbumin into the eye from day 15 to 26, 10 µL of each of N-acetyl-L-alanine, N-oleoyl-L-alanine, N-acetyl-L-tryptophan and N-oleoyl-L-tryptophan dissolved in phosphate-buffered saline to 100 µM was intraocularly administered 30 minutes and 12 hours later. On day 26, the clinical grading of conjunctival edema and flare (redness) was assessed using a portable slit lamp (Carl Zeiss, Oberkochen, Germany). The clinical grade of each test group was compared, and the result is shown in FIG. 7. Whereas a normal group showed almost no symptom of conjunctivitis and an OVA-challenged group showed an increased clinical grade of conjunctivitis (FIGS. 7A and 7B), the groups to which N-acetyl-L-alanine, N-oleoyl-L-alanine, N-acetyl-L-tryptophan or N-oleoyl-L-tryptophan M was intraocularly administered showed remarkably increased clinical grades (FIGS. 7A and 7B).

While the specific exemplary embodiments of the present disclosure have been described in detail above, it is obvious to those having ordinary knowledge in the art that such detailed descriptions are merely specific examples and the scope of the present disclosure is not limited by them. It is to be appreciated that the substantial scope of the present disclosure is defined by the appended claims and their equivalents.

## Claims

1. A pharmaceutical composition for preventing or treating asthma, rhinitis or conjunctivitis, comprising an N-acylamino acid or a pharmaceutically acceptable salt thereof as an active ingredient.

2. The pharmaceutical composition according to claim 1, wherein the N-acylamino acid is N-acylalanine or N-acyltryptophan.

3. The pharmaceutical composition according to claim 2, wherein the N-acylalanine is N-acetylalanine or N-oleoylalanine.

4. The pharmaceutical composition according to claim 2, wherein the N-acyltryptophan is N-acetyltryptophan or N-oleoyltryptophan.

5. A food composition for preventing or alleviating asthma, rhinitis or conjunctivitis, comprising an N-acylamino acid or a pharmaceutically acceptable salt thereof as an active ingredient.

6. The food composition according to claim 5, wherein the N-acylamino acid is N-acylalanine or N-acyltryptophan.

7. The food composition according to claim 6, wherein the N-acylalanine is N-acetylalanine or N-oleoylalanine.

8. The food composition according to claim 6, wherein the N-acyltryptophan is N-acetyltryptophan or N-oleoyltryptophan.
